(19) Europäisches Patentamt
European Patent Office
Office européen des brevets

(11) **EP 4 582 139 A1**

(12) **EUROPEAN PATENT APPLICATION**

(43) Date of publication:
**09.07.2025 Bulletin 2025/28**

(21) Application number: **25398001.5**

(22) Date of filing: **06.01.2025**

(51) International Patent Classification (IPC):
**A61N 1/378** *(2006.01)* **H02N 1/04** *(2006.01)*

(52) Cooperative Patent Classification (CPC):
**H02N 1/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.01.2024 PCT/EP2024/050228**

(71) Applicants:
• **Universidade Do Porto**
**4099-002 Porto (PT)**

• **INEB-Instituto Nacional De Engenharia Biomédica**
**4200-135 Porto (PT)**

(72) Inventor: **The designation of the inventor has not yet been filed**

(74) Representative: **Ferreira Pinto, Francisca**
**Garrigues IP, Unipessoal Lda.**
**Av. da República, 25-1°**
**1050-186 Lisboa (PT)**

(54) **A POWER GENERATION DEVICE FOR HARVESTING ENERGY THROUGH A FLUID-SOLID TRIBOELECTRIC NANOGENERATOR**

(57) This disclosure relates to a power generation device for harvesting energy through a fluid-solid triboelectric nanogenerator from a flow of body fluid flowing through or past the power generation device. The power generation device comprises a solid component, on which surface charges are inducible, at least one conductive component is comprised by the solid component or fixed to the solid component, and an output unit for outputting power. The at least one conductive component is electrically connected to the output unit. The solid component is configured to be brought into contact with a body fluid flowing through or past the power generation device by implanting the power generation device within a living organism or by bringing the power generation device into contact with or into proximity of a living organism.

Fig. 1

**Description**

Technical Field

The present disclosure relates to a power generation device for harvesting energy through a fluid-solid triboelectric nanogenerator. The disclosure also relates to a self-powered sensing device comprising such a power generation device, to a system comprising a self-powered sensing device, and to a wearable or implantable medical device. Moreover, this disclosure also relates to the use of a power generation device.

Technical Background

Cardiovascular diseases (CVDs) are one of the prominent causes of death worldwide. According to data provided by the WHO in 2022, the number of deaths associated with CVDs worldwide lies around 18 million, and this represents around 32% of all deaths. CVDs are unfortunately increasing all over the world due to sedentarism and aging and they are now one of the leading causes of disability adjusted life years lost.

It is pleasing that cardiac electronic devices (CEDs), such as pacemakers, defibrillators, or ventricular assist devices have been developed for managing CVDs. However, current generation implantable or wearable devices that require electrical power for functioning, have their limitations. In particular, they frequently require bulky batteries with a limited lifetime and introducing the risk of content leakage.

Several conditions such as atrial fibrillation, congenital heart diseases, coronary heart disease, or cardiomyopathy require CEDs to be treated, such as pacemakers, implantable cardioverter defibrillators (ICDs), cardiac resynchronization therapy (CRT) devices and left ventricular assist devices (LVAD). In approximately 85% of the overhaul procedures for these devices, which consist in the replacement of the whole device or the main device box (computer/-electronics and battery), is caused by battery depletion.

CEDs sometimes even need to be replaced in their entirety. This leads to additional health risks for patients, as such replacements may trigger infections, cause pain, lead to hematomas, and the like. This means that long-term therapy costs are not always kept under control to a satisfying degree.

There is, hence, a need for improved wearable and implantable electrically powered devices, and in particular, for improved wearable and/or implantable sensing systems that address at least one of the above-mentioned shortcomings.

Summary

One aspect of the present disclosure relates to a power generation device for harvesting energy through a fluid-solid triboelectric nanogenerator from a flow of body fluid flowing through or past the power generation device.

Triboelectric nanogenerators are energy harvesters that work on the basis of the triboelectric effect. The triboelectric effect refers to the phenomenon according to which dielectric materials (electrical insulators that can, e.g., upon electrical stimulation, be polarized, or that can, e.g., be spontaneously polarized) and/or their surfaces become electrically charged when they are in contact with another material of opposite tribopolarity. This becoming charged due to contact with a charged substance is also referred to as contact electrification. A surface's tribopolarity is determined by the material's tendency to lose or gain electrons, leading to positive or negative charges, respectively.

When using solid-solid triboelectric generators (solid-solid TENGs), two materials that are in contact with each other will move relatively to each other due to external mechanical movements, and the oppositely charged surfaces will change periodically, from the material with higher energy to the one with the lower energy of occupied surface state. This induces a potential difference between the electrodes attached to the back of the materials and connected by an external circuit - electrostatic induction. An electrostatic equilibrium is maintained by having the free electrons move back and forth. This produces a power output. The so-called single electrode mode (SE-mode) of the TENGs may have the simplest configuration, according to which a sole electrode is grounded, and the contact separation or sliding of triboelectric surfaces establishes a current flowing between the ground and another electrode.

The term fluid-solid triboelectric nanogenerator (also abbreviated as "Flu-TENG"), as used herein, refers to a nanogenerator that uses a fluid and a solid as the two materials for the triboelectric layers. Similarly to the traditional solid-solid contact TENGs, as the liquid moves over the solid surfaces, opposite charges are generated in both materials.

The flowing through the power generation device of body fluid refers to a configuration in which the power generation device comprises at least one pathway itself, through which the body fluid flows.

The flowing past the power generation device of body fluid refers to a configuration in which the body fluid does not flow into an inner space of the power generation device itself, but flows past it, for example, by flowing along an outer surface of the power generation device.

The power generation device may comprise a solid component, on which surface charges are inducible. The induction of charges may in fact be on the solid component itself or on a conductive component provided on the solid component.

In particular, the power generation device may comprise at least one conductive component. The conductive component may be comprised by the solid component. In other words, the solid component may be a conductor or comprise one or several conductors. One or several conductive components may be fixed to the solid component. A conductive material may be sprayed, sprinkled, or otherwise applied to the solid component.

The power generation device may comprise an output unit for outputting power. The output unit may be configured to output power to a consumption unit. However, the output unit may just output power that may be detected by an internal or an external detection unit. The detection unit may be remotely located.

The at least one conductive component may be electrically connected to the output unit. This may enable the power output.

The solid component may be configured to be brought into contact with a body fluid flowing through or past the power generation device by implanting the power generation device within a living organism or by bringing the power generation device into contact with or into proximity of a living organism. In other words, when the power generation device is in position, that is, after having implanted it in a living organism or after having brought it into contact therewith or into proximity thereof, the solid component may be in contact with a body fluid following through it or past it.

This may enable using the body fluid (that may be in motion) as one of the two parts of a fluid-TENG. This way, energy may be harvested from a moving body fluid, such as, for example, blood, urine, sweat, filtered blood, lymphatic fluid, cerebrospinal fluid, serum, bile, blood plasma, saliva, synovial fluid, menstrual fluid, or tear fluid.

The power generation device in accordance with the present disclosure may allow outputting power to a medical device that is implanted into a living organism or is brought into contact or into proximity with a living organism. This may offer the possibility of conveniently powering devices such as cardiac electronic devices and/or sensors, without the need of changing batteries and the like. This may contribute to monitoring health status, promoting longevity, reducing inconvenience, and reducing risks associated with surgical interventions associated with maintenance such as battery changes of medical devices. The latter may in particular prevent infection risks, pain, and/or hematomas in patients. In addition, long term therapy costs for patients may be lowered.

In addition, the power generation device in accordance with the present disclosure may be very small, thus offering advantages of over conventional powering devices used inside or outside of living organisms. Moreover, the power generation device in accordance with the present disclosure may mitigate the risks associated with toxic content leakage from conventional power generation devices.

The described fluid-solid-TENG may have advantages over solid TENGs, in particular, such as reduction of mechanical wear, since the latter's durability is highly affected by wear abrasion, compromising long-term performance and performance stability. Moreover, there may be less influence from external factors and, in the case of a solid TENG, it may be hard to assure the proper contact between the triboelectric layers' materials. In contrast, a body fluid has the capability of adjusting its shape, thus promoting better contact between the two electrodes of a fluid-solid TENG. The electric outputs of the power generation device in accordance with the present disclosure may thus be improved.

The solid component may comprise at least one polymer and/or at least one metal. The at least one metal may be a conductive component as referred to above.

In particular, the solid component may comprise one or several materials selected from the group consisting of: expanded polytetrafluoroethylene (ePTFE), polyethylene terephthalate, poly(L-lactic acid) (PLLA), poly-2-hydroxyethyl methacrylate graphene oxide (pHEMA/GO), polyethylene glycol graphene oxide (PEG/GO), other polymers, such as tubular polymers, silicon, polyurethane, metals, such as titanium, aluminum, gold, platinum, nickel, copper and/or stainless steel, and metal alloys, such as nitinol, cobalt-chromium alloys, magnesium alloys, brass.

The solid component may comprise a lumen for letting a body fluid flow therethrough. This is an example of a configuration of the power generation device for letting a body fluid through the power generation device.

The solid component may comprise a surface (in particular: an inside surface) facing the lumen, wherein charges may be inducible on the surface (the inside surface). To this end, the surface may comprise conductive elements or be made of a conductive material.

For example, an inside surface of the solid component may have an annular shape and extend partially or fully around the lumen. This may be a configuration when using the power generation device as a part of an artificial graft and the like.

At least a part of the solid component may be hollow and configured to let a body fluid pass therethrough. This is an example of a configuration of the power generation device for letting a body fluid through the power generation device. The hollow configuration may in particular provide the possibility for body fluid to flow into the power generation device and to flow out of the power generation device after energy has been harvested therefrom.

The power generation device may comprise a part that is at least partially tube-shaped. For example, if the power generation device forms part of an artificial vessel, the artificial vessel may be a tube-shaped part.

An inner diameter of the at least partially tube-shaped part may be in the range of from 0.1 mm to 40 mm.

An inner diameter of the at least partially tube-shaped part may be in the range of from 0.5 mm to 30 mm, or from 1 mm to 20 mm, or from 1.1 mm to 10 mm, or from 1.2 mm to 6.0 mm, or from 1.3 mm to 2.0 mm.

In each of the mentioned cases, the part may be not only partially, but fully tube-shaped.

A wall thickness of the at least partially tube-shaped part may be in a range of from 10 $\mu$m to 10 mm, optionally, from 15 $\mu$m to 1000 $\mu$m, from 50 $\mu$m to 700 $\mu$m, or from 70 $\mu$m to 600 $\mu$m.

This disclosure also relates to a power generation device for harvesting energy through a fluid-solid triboelectric nanogenerator from a flow of body fluid flowing through or past the power generation device, wherein the power generation device may comprise at least one conductive component configured to be brought into contact with a solid component that is in contact with a body fluid flowing through or past the power generation device by implanting the power generation device within a living organism or by bringing the power generation device into contact with or into proximity of a living organism. In other words, the solid component is not necessarily a part of the power generation device. For example, a natural vessel in a living organism may form a solid component of a fluid-solid-TENG, wherein metal may be sprayed (or otherwise deposited) onto the vessel, with the metal particles being a conductive component as mentioned above.

The power generation device may comprise an output unit for outputting power. The power generation device may be configured to output power to a consumption unit. In other cases, the power may not be output to a consumption unit, but may, for example, be monitored for detection purposes.

The at least one conductive component may be electrically connected to the output unit.

In combination with any of the mentioned features described above (any aspect), the at least one conductive component may be configured to be partially or fully wrapped around the solid component (for example before the power generation device is placed in a position in which it will serve the purpose of harvesting energy), or it may be partially or fully wrapped around the solid component (in the case of some examples, this will always be the case, whereas in the case of other examples, this will be the case once the power generation device has been brought into the position in which it will serve the purpose of harvesting energy).

In addition or alternatively thereto, the at least one conductive component may be configured to be physically and/or chemically bound to the solid component, or it may be physically and/or chemically bound to the solid component (in the case of some examples, this will always be the case, whereas in the case of other examples, this will be the case once the power generation device has been brought into the position in which it will serve the purpose of harvesting energy).

The at least one conductive component may be configured to be painted, sprayed, deep coated, sputtered, or glued onto the solid component, or it may be painted, sprayed, deep coated, sputtered, or glued onto the solid component (in the case of some examples, this will always be the case, whereas in the case of other examples, this will be the case once the power generation device has been brought into the position in which it will serve the purpose of harvesting energy).

The at least one conductive component may be configured to be directly or indirectly exposed to the body fluid flowing through or past the power generation device upon the power generation device being implanted within a living organism or being brought into contact with or into proximity of a living organism.

By being directly exposed, what is meant, is that the at least one conductive component is touched by the body fluid. By being indirectly exposed, what is meant, is that the least one conductive component is not touched, as there is something in-between. However, electrical charges may nevertheless be induced due to the passing body fluid.

The power generation device may comprise a conductive wire electrically connecting the at least one conductive component and the output unit.

The at least one conductive component may comprise at least one element selected from the group consisting of: silver, carbon-based materials, such as graphite, graphene, or carbon nanotubes, PEDOT:PSS (poly(3,4-ethylenedioxythiophene): polystyrene sulfonate), copper, magnesium, silver nanoparticles, platinum, gold, tantalum, titanium, silicone, iridium, iridium oxide, indium tin oxide, niobium, yttrium barium copper oxide, and magnesium diboride, bronze, brass, iron and tungsten.

The at least one conductive component may have a length in a range of from 10 nm to 50 cm, optionally, from 100 nm to 50 cm, from 1 $\mu$m to 50 cm, from 10 $\mu$m to 20 cm, from 0.5 cm to 10 cm, or from 1 cm to 9 cm.

The power generation device may comprise a plurality of conductive elements which are configured to be directly or indirectly exposed to the body fluid flowing through or past the power generation device and which are electrically connected to the output unit.

This disclosure also relates to a self-powered sensing device comprising a power generation device in accordance with the present disclosure (comprising any one or several of the features described above).

The self-powered sensing device in accordance with the present disclosure may allow outputting performing sensing after having been implanted into a living organism or having been brought into contact or into proximity with a living organism. This may offer the possibility of conveniently performing sensing operations, without the need of changing batteries and the like. This may contribute to monitoring health status, promoting longevity, reducing inconvenience, and reducing risks associated with surgical interventions associated with maintenance such as battery changes of medical devices, as the self-powered sensing device may not need any battery at all. The latter may in particular prevent infection risks, pain, and/or hematomas in patients. In addition, long term therapy costs for patients may be lowered.

In addition, the self-powered sensing device in accordance with the present disclosure may be very small, thus offering advantages of over conventional sensing devices used inside or outside of living organisms. Moreover, the self-powered sensing device in accordance with the present disclosure may mitigate the risks associated with toxic content leakage from conventional sensing devices.

In particular, the at least one conductive component of the self-powered sensing device may have a length in a range of from 10 nm to 40 cm, or from 15 nm to 20 cm, or from 20 nm to 10 cm.

The self-powered sensing device may be configured to be implanted in, on, or around a blood vessel, a fistula, bronchi, intestines, nephrons, a ureter, an Eustachian tube, and/or a Fallopian tube.

The self-powered sensing device may be configured to transmit data to an external device. This function may be used to alert patients or healthcare providers and/or to integrate the self-powered sensing device in the Internet of Medical Things (IoMT).

The self-powered sensing device may be configured to wirelessly transmit data to an external device. This function may be used to alert patients or healthcare providers and/or to integrate the self-powered sensing device in the Internet of Medical Things (IoMT).

The self-powered sensing device may be configured to execute a function in response to sensing a predetermined signal. The function may be releasing a drug. For example, a predetermined amount of a drug may be released, or a predetermined quantity may be released over a time period.

The self-powered device may act as a smart device for theranostics and/or treatment. For example, a drug could be released in response to the sensing of a predetermined capacity. Alternatively, whenever it is sensed that a vascular graft is occluding, a drug to dissolve the clot could be released.

This disclosure also relates to a system comprising the self-powered sensing device in accordance with the present disclosure comprising any one or several features described above (including any one or several features of the power generation device in accordance with the present disclosure).

The system may be configured to measure a potential difference between a first potential measured at a first point in time and a second potential measured at a second point in time. The first potential and the second potential may be measured at the same location or at different potentials. The system may be configured to measure further potentials at further points in time at the (with respect to at least one of the other measured locations) same location or at respective different locations.

The system may be configured to detect a flow obstruction, such as a blood clot, biofilms and encrustation, in function of the potential difference between the first potential and the second potential. In particular, a flow obstruction, a blood clot, biofilms and/or encrustation may be detected earlier on. Therefore, the system may allow for preemptively reacting to an impending problem before it manifests itself with any symptoms.

For example, a blood flow rate may be highly dependent on the type of vessel and its location in a living organism, since the blood flow rate may vary with a difference in pressure ($\Delta P$) and inversely to a vasculature resistance (R), which consists of a resistance that must be overcome so as to force the flow of blood along the circulatory system. Relating these variables, the blood flow rate (Q) can be defined by the Hagen-Poiseuille equation, that can be further applied for other types of fluids, as

$$Q = \frac{\Delta P}{R} = \frac{\pi \Delta P r^4}{8 \eta \lambda}$$

where r is the radius of the vessel, $\eta$ the blood's viscosity and $\lambda$ is the vessel's length. The same may be applied for an implanted vascular graft. Considering that fluid-solid-TENGs may be sensible to flow rate variations, a body fluid flow rate may vary with location and also, pathological conditions that affect it, such as blood pressure and viscosity alterations, may affect the obtained triboelectric outputs. As such, these factors may be considered during the fluid-solid-TENGs design, depending on the envisaged application.

The detection in function of the potential difference may be a detection in function of whether the potential difference is larger than a first preset threshold value and/or is smaller than a second preset threshold value.

The system may be configured to measure a potential difference between a first electrode and a second potential measured at a second electrode. The system may be configured to measure potentials at several positions, e.g., using a micro array, and may be configured to measure potential differences between several potentials.

The system may be configured to measure a current flowing, a voltage, and/or a power density of a current flowing between the first electrode and the second electrode (or between two or more electrodes, or may be configured to perform several measurements at different positions).

The system may be configured to detect a flow obstruction, such as a blood clot, biofilms and/or encrustation, in function of the potential difference between the first electrode and the second electrode.

The system may be configured to detect a flow obstruction, such as a blood clot, biofilms and/or encrustation, in function of whether the potential difference exceeds a first preset threshold value and/or is lower than a second preset threshold value.

The system may be configured to detect a flow obstruction, such as a blood clot, biofilms and/or encrustation, in function of whether a difference between a potential difference at a first point in time and a potential difference at a second point in time exceeds a second preset threshold value.

This disclosure also relates to a wearable or implantable medical device comprising a power generation device in accordance with the present disclosure (comprising any one or several of the features described above) and/or a self-powered sensing device in accordance with the present disclosure (comprising any one or several of the features described above).

The wearable or implantable medical device may be selected from the following list: a catheter, an endovascular stent, a tubular medical device, a drainage tubes, a chest tube, an abdominal drain, a feeding tube, a ventricular shunt, a vascular graft, a decellularized vessel, an autologous graft, a xenograft, an allograft, a collagen-based graft, a decellularized organ, and a component of an extracorporeal circuit machine, e.g., including a tube or a tubing.

The wearable or implantable medical device may comprise a consumption unit. The output unit of the power generation device may be connected to the consumption unit.

The consumption unit may be selected from the following list: a sensor, a medical electronic device, such as a cardiac electronic device, a pacemaker, an implantable cardioverter defibrillator, a cardiac resynchronization therapy device, an artificial heart, an artificial organ, and a left ventricular assist device.

The wearable or implantable medical device may be an implantable vascular graft comprising a tubular body, the tubular body being comprised by the solid component or being the solid component of the power generation device.

This disclosure also relates to the use of the power generation device (or of the self-powered sensing device or the system) in accordance with the present disclosure (comprising any one or several of the features described above) for outputting electrical power and/or as a sensing device by monitoring a power output by the power output unit.

The monitoring of the power output may comprise determining a variation of flow of the body fluid over time or between at least two locations or an adsorption of one or several biocomponents, such as a blood clot, a biofilm, bacteria, or proteins, along the power generation device.

The power generation device (or of the self-powered sensing device or the system) may be used for sensing a variation in a flow of a body fluid such as blood, urine, filtered blood, lymphatic fluid, cerebrospinal fluid, serum, bile, blood plasma, saliva, synovial fluid, menstrual fluid, or tear fluid.

The power generation device (or of the self-powered sensing device or the system) may be used for powering a wearable or implantable electronic medical device.

Additional advantages and features of the present disclosure, that can be realized on their own or in combination with one or several features discussed above, insofar as the features do not contradict each other, will become apparent from the following description of particular embodiments.

Brief Description of the Drawings

For a better understanding of the present disclosure and to show how the same may be carried into effect, reference will now be made, by way of example only, to the accompanying drawings, in which:

The description is given with reference to the accompanying drawings, in which:

Fig. 1      depicts an embodiment of a power generation device in accordance with the present disclosure;

Fig. 2      depicts a transversal sectional view of a power generation device in accordance with the present disclosure;

Fig. 3A     depicts a first possible charge generation mechanism for a fluid-solid TENG that is part of the power generation device of Fig. 2;

Fig. 3B     depicts a second possible charge generation mechanism for a fluid-solid TENG that is part of the power generation device of Fig. 2;

Fig. 4      depicts a transversal sectional view of a power generation device in accordance with the present disclosure;

Fig. 5A     depicts a first possible charge generation mechanism for a fluid-solid TENG that is part of the power generation device of Fig. 4; and

Fig. 5B     depicts a second possible charge generation mechanism for a fluid-solid TENG that is part of the power generation device of Fig. 4.

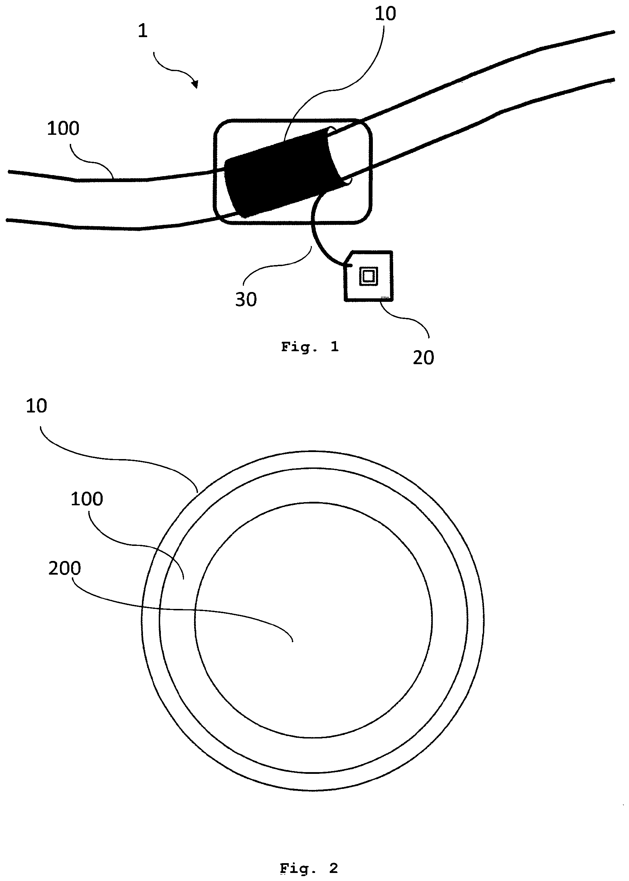

Fig. 1 depicts an embodiment of a power generation device 1 in accordance with the present disclosure. The power generation device 1 is in this case configured to harvest energy from a flow of blood in a living organism.

Fig. 1 depicts a native vessel or a vascular graft 100 in a living organism. The power generation device 1 is shown in a state in which it has been implanted in the living organism.

The encircled area shows the power generation device 1 that harvests energy through a fluid-solid triboelectric nanogenerator from a flow of body fluid flowing through the power generation device 1. While the body fluid is in this case flowing through the power generation device 1, the body fluid may instead, in the case of other embodiment, flow past the power generation device 1 (e.g., along an outer surface of the power generation device 1).

Fig. 2 shows a transversal sectional view of the power generation device 1 depicted in Fig. 1 and, in particular, depicts the setup of the fluid-solid TENG (TENG stands for triboelectric nanogenerator).

A body fluid 200 flows through the power generation device 1. In the case of this embodiment, the body fluid 200 is blood flowing through the native vessel of vascular graft 100. However, in other embodiments, the body fluid 200 may be different. It may, for example, be urine, filtered blood, lymphatic fluid, cerebrospinal fluid, serum, bile, blood plasma, saliva, synovial fluid, menstrual fluid, or tear fluid.

The body fluid 200 in the case of this embodiment flows either in the native vessel 100 (variant A) or in the vascular graft (variant B). In so far, both variants A and B are embodiments that are in accordance with what is depicted in Figs. 1 and 2.

The native vessel 100 or the vascular graft 100 is the solid component of the fluid-solid TENG. In the case of the native vessel 100, the solid component is not part of the power generation device 1 but is a part of the living organism. In contrast, in the case of the vascular graft 100, the solid component may be a component of the power generation device 1.

Surface charges are inducible on the native vessel 100 (variant A) or on the vascular graft (variant B).

An electrode, as a conductive component 10, is wrapped (partially or fully) around the native vessel 100 or the vascular graft 100. This is depicted both in Figs. 1 and 2.

In the case of the embodiment of Fig. 1 and 2, the power generation device 1 also comprises a power management unit, as an example of an output unit 20 for outputting power. The output unit 20.

In addition, the embodiment of Figs. 1 and 2 comprises a wire 30 that electrically connects the conductive component 10 with the output unit 20.

As becomes apparent from Figs. 1 and 2, the native vessel 100 (variant A) or the vascular graft 100 (variant B), as examples of the solid component, are in contact with the body fluid 200 (in this case: blood) flowing through the power generation device 1.

Blood flow causes a relative movement and, hence, a friction, between blood constituents (in particular, plasma, blood cells, and salts) on the vessel walls or vascular grafts (upon patient implantation), and that this leads to triboelectrification of all the involved components. In particular, the membrane of red blood cells (erythrocytes) has unique characteristics that may allow the generation of high levels of triboelectricity.

The vascular graft 100 in the case of this example comprises expanded polytetrafluoroethylene (ePTFE). However, in the case of other embodiments, the material may be different. More generally, the vascular graft 100 may, for example, comprise a one or several materials selected from the group consisting of: expanded polytetrafluoroethylene (ePTFE), polyethylene terephthalate, poly(L-lactic acid) (PLLA), poly-2-hydroxyethyl methacrylate graphene oxide (pHEMA/GO), polyethylene glycol graphene oxide (PEG/GO), other polymers, such as tubular polymers, silicon, polyurethane, metals, such as titanium, aluminum, gold, platinum, nickel, copper and/or stainless steel, and metal alloys, such as nitinol, cobalt-chromium alloys, magnesium alloys, brass.

The native vessel 100 or the vascular graft 100 is hollow and comprises a lumen through which the blood flows in the case of this example. The inside surface of the native vessel 100 or the vascular graft 100 faces the lumen, and charges are inducible thereon.

The electrode, as an example of a conductive component 10 is in this case tube-shaped. However, other shapes (e.g., partially tube-shapes or completely different shape) are possible as well.

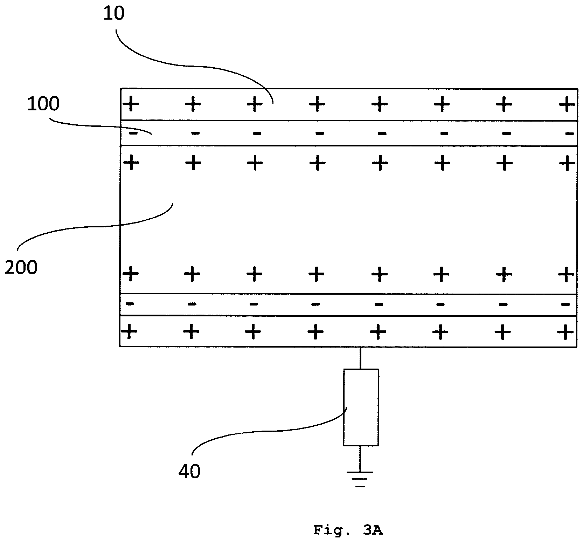

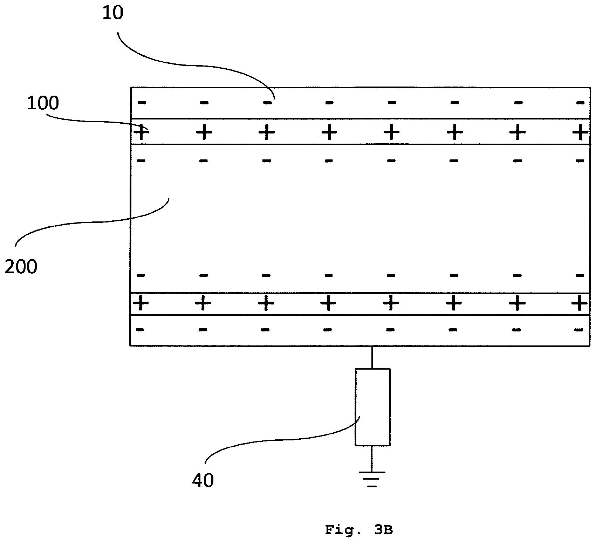

Fig. 3A and 3B depict sectional views of the power generation device 1 of Fig. 2 in a direction perpendicular to the sectional view of Fig. 2. Figs. 3A and 3B namely show sectional views in a direction perpendicular to the length direction of the natural vessel 100 (variant A) or the vascular graft 100 (variant B).

Figs. 3A and 3B illustrate two possible charge generation mechanisms for the fluid-solid TENG that is part of the power generation device 1 depicted in Figs. 1 and 2.

In the case of Fig. 3A, a positively charged body fluid is flowing through the power generation device 1. Consequently, an excess of negative charges is induced or previously charged on the solid component, which may in the case of the depicted embodiment in particular be the natural vessel 100 (variant A) or the vascular graft 100 (variant B). This in turn implies that and an excess of positive charges is induced on the electrode, as an example of a conductive component 10 that forms part of the power generation device 10.

In contrast, in the case of Fig. 3B, a negatively charged body fluid is flowing through the power generation device 1. Consequently, positive charges are induced on the solid component, which may in the case of the depicted embodiment in particular be the natural vessel 100 (variant A) or the vascular graft 100 (variant B). This in turn implies that negative charges are induced on the electrode, as an example of a conductive component 10 that forms part of the power generation device 10.

Both in Figs. 3A and 3B, a general load 40 is depicted, as an example for where an output unit is to be connected or, depending on the embodiment, a consumption unit. For example, a wire may be connected to the fluid-solid TENG for harvesting power.

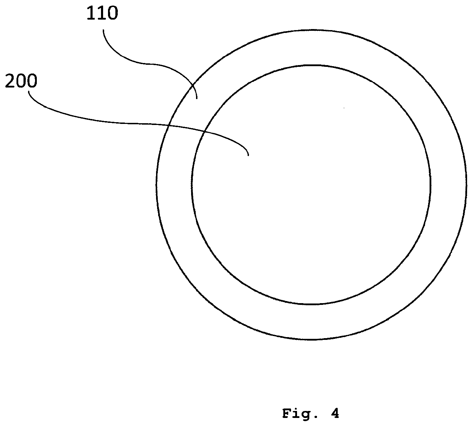

Fig. 4 shows a transversal sectional view of an embodiment of a power generation device 1 and, in particular, depicts the setup of a fluid-solid TENG (TENG stands for triboelectric nanogenerator).

The embodiment of Fig. 4 is a power generation device 1 wherein the solid component 110 is a vascular graft 110 made of synthetic material that includes metal particles, as an example of a conductive component.

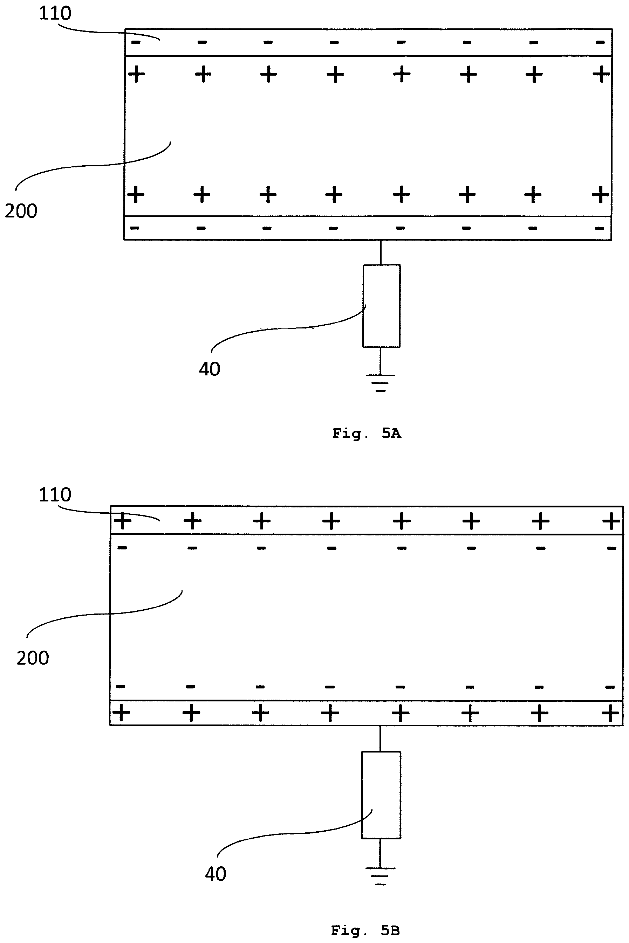

Figs. 5A and 5B illustrate two possible charge generation mechanisms for the fluid-solid TENG that is part of the power generation device 1 depicted in Fig. 4.

In the case of Fig. 5A, a positively charged body fluid is flowing through the power generation device 1. Consequently, negative charges are induced on the solid component 110.

In contrast, in the case of Fig. 5B, a negatively charged body fluid is flowing through the power generation device 1. Consequently, positive charges are induced on the solid component 110.

Both in Figs. 5A and 5B, a general load 40 is depicted, as an example for where an output unit is to be connected or, depending on the embodiment, a consumption unit. For example, a wire may be connected to the fluid-solid TENG for harvesting power.

<u>Examples</u>

Exemplary power generation devices were manufactured to demonstrate the use of the flow of a body fluid in native vessels and (bio)materials, in particular blood in arteries and vascular grafts, to generate electrical energy from a living body source.

Fluid-solid TENGs were developed with synthetic vascular grafts as solid-phases, in particular ePTFE, commercially available, or pHEMA/GO, produced in the lab, as well as with native arteries. Different fluids were tested, namely PBS, plasma and blood. As electrodes, silver, carbon and PEDOT:PSS conductive inks and copper tape were tested regarding their stability in the solid phase (washing tests and SEM), efficiency (electrical outputs) and biocompatibility (towards fibroblast cells (HFF-1)).

Exemplary assembly of conductive ink electrodes on ePTFE was done by brush painting, as stable coatings were observed and there was no infiltration to the interior of the vascular grafts. Silver showed to be more stable for pHEMA/GO vascular graft and arteries. Charging 1 and 100 nF capacitors, it was evaluated, on the basis of their saturation, how fluid-solid TENGs performed. High outputs were achieved with plasma as the fluid-phase (23.6 V), when compared to PBS (17.3 V), for which the results were closer to using blood (14.2 V). It was observed that the performance of fluid-solid TENGs depends on a flow rate of the body fluid. For example, an increase of 100 mL/min in a flow rate was observed to be associated with an output that was higher by around 10 V. Different electrodes were tested, and an output of 17.3 V was, for example, achieved with silver, 12.9 V with carbon, and 10.4 V with PEDOT:PSS. The output was observed to linearly depend on the electrode length, with an observed increase of 30 V passing from 0.5 to 10 cm of a silver electrode. The different solid-phases analyzed showed triboelectric properties, having obtained at least 10.5 V with ePTFE and 14.5 V with pHEMA/GO vascular grafts, and 8 V with the artery. These results are influenced by the material's wall thickness, whose increase decreases the output, as does its inner diameter. Finally, it was shown the Flu-TENGs potential as a narrowing sensor, as perturbation of flow induced by 30% narrowing of an ePTFE vascular graft led to a perturbation in the electrical output of 8 V. Therefore, changes in the electrical signal could be associated with narrowing levels, for their detection.

Exemplary expanded polytetrafluoroethylene (ePTFE) vascular grafts were produced that could be used for peripheral bypass surgery. In particular, exemplary vascular grafts had different inner diameters (ID) and wall thicknesses (WT), namely, for example, 1.5 mm ID and 100 $\mu$m WT, 4 mm ID and 500 $\mu$m WT, 4.5 ID mm and 75 $\mu$m WT and 6 ID mm and 152 $\mu$m WT.

In addition, vascular grafts made of poly(2-hydroxyethyl methacrylate), an FDA approved hydrogel, and graphene oxide (pHEMA/GO) with 4 mm ID and 1 mm WT were used to observe the triboelectric effect. For manufacturing purposes, 2-hydroxyethyl methacrylate (HEMA) monomers were polymerized in situ by adding 1.5% (v/v) of a 1:1 mixture of 40% ammonium persulfate (APS) and 15% sodium metabisulfite (SMB), in the presence of 1% (m/v) of GO (prepared by oxidation graphite by

modified Hummer's method) and 3% (v/v) of crosslinking agent tetraethylene glycol dimethacrylate (TEGDMA). 3D tubes were produced resorting to a plastic tube mold and inner glass rod of 6 and 4 mm diameter, respectively. The hydrogels were then removed from the molds, after an overnight resting, and rinsed with distilled water.

Moreover, native coronary heart arteries were studied as solid components.

For conductive components, different conductive inks were studied, for example, namely silver (VFP ink technologies, SE-001), carbon (Elantas, Bectron® GP 9553) and poly(3,4-ethylenedioxythiophene):polystyrene sulfonate (PEDOT:PSS, Heraeus, Clevios TM S V4 STAB, 81098168). They were applied to a vessel or graft by brush painting. A copper wire was attached to all conductive inks to allow the connection to the acquisition set up. In the case of synthetic vascular graft, the inks were cured for 10 minutes under 130 °C to achieve maximum conductivity, as recommended by the inks' suppliers, while for native vessels, to avoid vessel damaging due to the high temperature, they were left drying at room temperature for 30 minutes. Besides conductive inks, copper tape was also tested as electrode. Copper tape was wrapped in the different solid-phases. For silver conductive ink, it was tested the influence of different electrode lengths (0.5, 1, 2, 4, 6, 8 and 10 cm) and designs in the achieved triboelectric outputs.

The following experimental data was obtained (see table 1):

Table 1

| Fluid-solid TENG component | Parameter | Parameter Specification | $V_{out}$ [V] |
|---|---|---|---|
| Fluid | Type | PBS | 17.3 |
| | | Plasma | 23.6 |
| | | Blood | 14.2 |
| | Flow-rate | 60 rpm | 15.3 |
| | | 100 rpm | 24.6 |
| Electrode | Type | Silver | 17.3 |
| | | Carbon | 12.9 |
| | | PEDOT:PSS | 10.4 |
| | Length | 0.5 cm | 7.6 |
| | | 1 cm | 17.0 |
| | | 2 cm | 18.9 |
| | | 4 cm | 26.2 |
| | | 6 cm | 29.4 |
| | | 8 cm | 38.5 |
| | | 10 cm | 47.6 |
| | | ePTFE vascular graft | 10.5 |
| | | pHEMA/GO vascular graft | 14.5 |
| | | Umbilical cord artery | 8.0 |
| | Wall thickness | 75 μm | 13.5 |
| | | 500 μm | 10.5 |
| | Diameter | 1.5 mm | 20.3 |
| | | 6 mm | 15.3 |

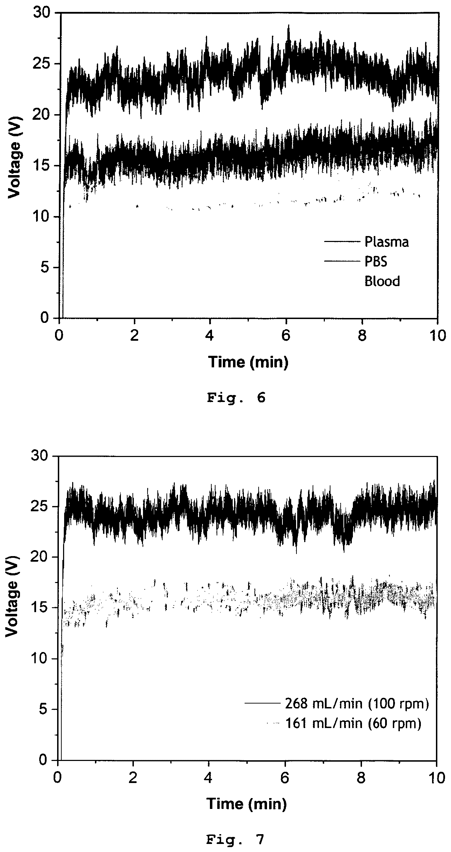

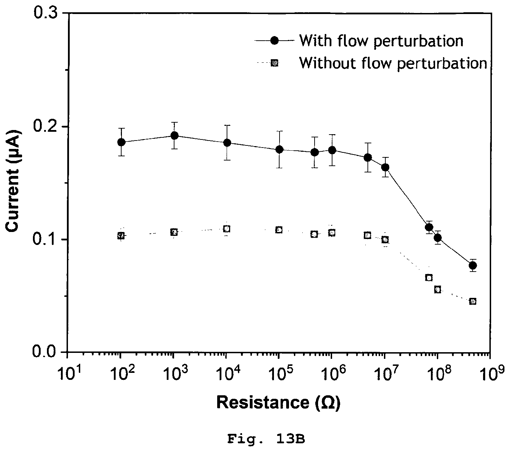

Figure 6 to 13B show experimental evidence corresponding to Table 1 that was obtained studying various embodiments of fluid-solid TENGs in accordance with the present disclosure. Figs. 6-13A show measured voltage outputs in function of time. Fig. 13B shows a current output in function of the resistance.

Fig. 6 illustrates experimental evidence of the charging of 1nF capacitors using fluid-solid TENGs with different fluids flowing therethrough. Three different fluids were used as the respective fluid stage of the TENGs: PBS (phosphate-buffered saline), plasma, and blood, and the figures shows three corresponding curves illustrating the respective measured voltage over time

Fig. 7 illustrates experimental evidence of the charging of 1nF capacitors using fluid-solid TENGs combined with different flow densities of the fluid flowing therethrough. Namely, the flow density of 161 mL/min of fluid was compared with 268 mL/min. This is an indicator for the change in charging capacity in function of perturbed vs. unperturbed flows through blood vessels, and the like. The tested fluid-solid TENG comprised a solid phase, consisting of ePTFE and with an inner diameter of 6 mm and with a wall thickness of 152 $\mu$m, and a silver electrode with a length of 2 cm. From the observation of results, one finds that the outputs increase with an increase in flow rate.

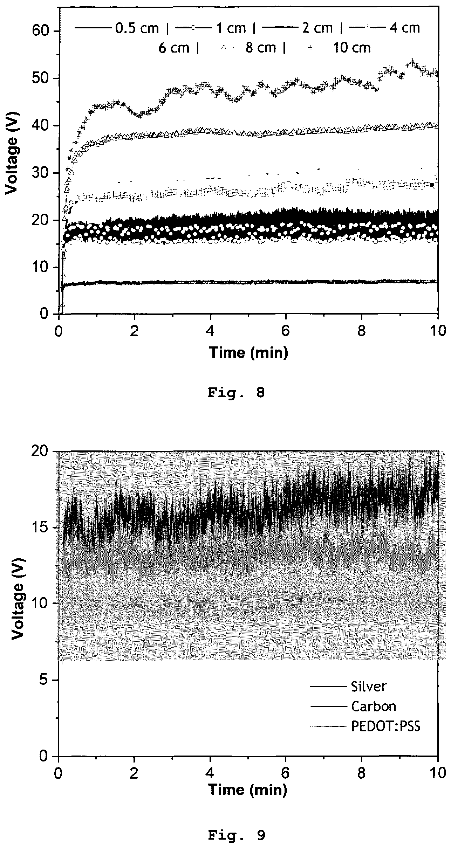

Fig. 8 illustrates experimental evidence of the charging of 1nF capacitors with fluid-solid TENGs having different electrode lengths. Electrode lengths of 0.5 cm, 1 cm, 2 cm, 4 cm, 6 cm, 8 cm, and 10 cm were tested. The tested fluid-solid TENG comprised a solid phase, consisting of ePTFE and with an inner diameter of 1.5 mm and with a wall thickness of 100 $\mu$m, and with a PBS fluid phase (with a flow density of 161 mL/min).

Fig. 9 illustrates experimental evidence of the charging of 1nF capacitors with fluid-solid TENGs having different types of electrodes (different electrode materials). In particular, silver electrodes, carbon electrodes, and PEDOT:PSS electrodes were used. The tested fluid-solid TENG comprised a solid phase, consisting of ePTFE and with an inner diameter of 1.5 mm and with a wall thickness of 100 $\mu$m, and with a PBS fluid phase (with a flow density of 161 mL/min).

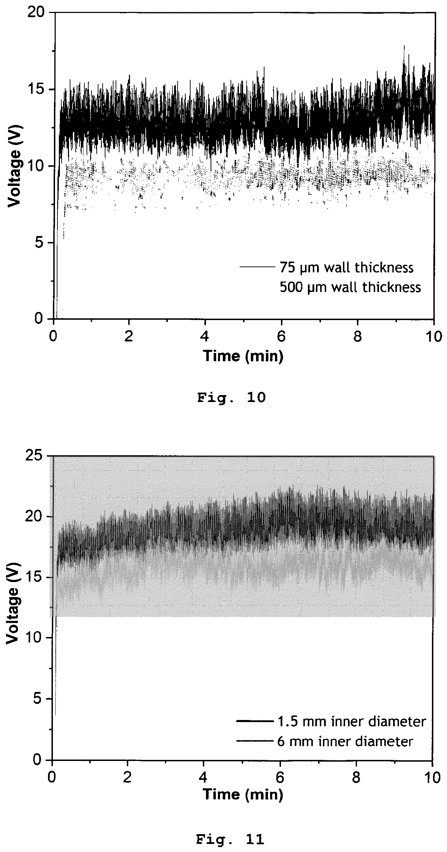

Fig. 10 illustrates experimental evidence of the charging of 1nF capacitors with fluid-solid TENGs having different solid phase wall thicknesses. Namely, the wall thickness of 75 $\mu$m (an inner diameter of 4.5 mm) was compared with the wall thickness of 500 $\mu$m (and inner diameter of 4 mm). The fluid phase was PBS (with a flow density of 161 mL/min), and the electrode was a 2 cm length silver electrode. From the observation of results, one finds that higher outputs were achieved when solid-phase's wall thickness was reduced.

Fig. 11 illustrates experimental evidence of the charging of 1 nF capacitors with fluid-solid TENGs having different inner diameters. Namely, an inner diameter of 1.5 mm (and a wall thickness of 100 $\mu$m) was compared with an inner diameter of 6 mm (and a wall thickness of 152 $\mu$m). The tested fluid-solid TENG comprised a solid phase, consisting of ePTFE, the fluid phase was PBS (with a flow density of 161 mL/min). The figures shows that the output increases with a smaller diameter.

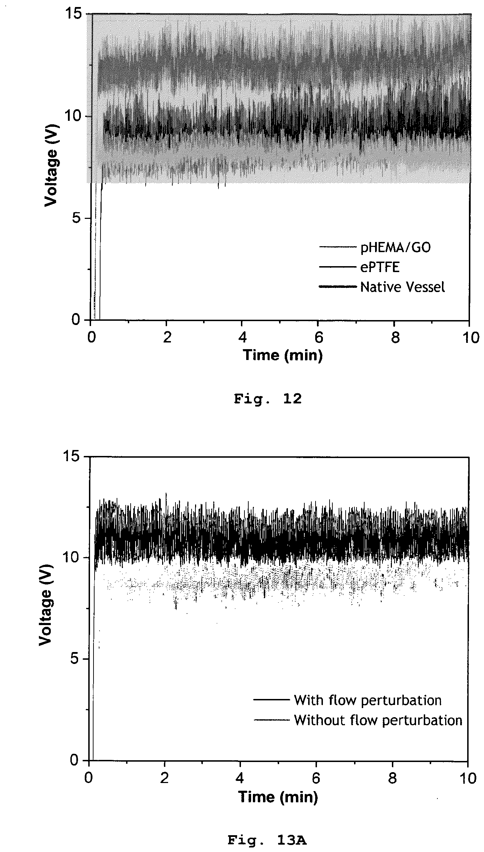

Fig. 12 illustrates experimental evidence of the charging of 1nF capacitors with fluid-solid TENGs having different solids phases. The tested solid phases were ePTFE (4 mm of inner diameter and 500 $\mu$m of wall thickness), PHEMA/GO (4 mm of inner diameter and 1 mm of wall thickness), and a natural artery (an umbilical cord). The tested fluid-solid TENG had a PBS fluid phase (with a flow density of 161 mL/min) and a 2cm length silver electrode.

Fig. 13A illustrates experimental evidence of the charging of 1nF capacitors with fluid-solid TENGs comparing a flow without perturbation and a flow with perturbation (the flow perturbation was a 30% narrowing of the solid phase resulting in a corresponding higher resistance). The tested fluid-solid TENG comprised a solid phase, consisting of ePTFE and with an inner diameter of 4 mm and with a wall thickness of 500 $\mu$m, and with a PBS fluid phase (with a flow density of 161 mL/min), as well as a 2 cm length silver electrode.

Fig. 13B illustrates a measured current for the fluid-solid TENGs, to which Fig. 13A relates, in function of the resistance.

By comparing the acquired electrical signals of Figs. 13A and 13B, it is possible to observe an increase in current outputs for all tested resistances, upon 30% narrowing of the solid-phase. Moreover, voltage outputs are also increased. Thus, fluid-solid TENGs can be used very well for detecting the narrowing of vascular grafts or natural vessels, as the corresponding electrical signals are good indicators for occlusions and also for the degree of occlusion.

Throughout this text, when it is stated that a structural component (e.g., a system, a device, etc.) is configured to do something, this may be understood to express also that the system or device is doing it (e.g., the statement that a component is configured to sense is to be understood also as a disclosure of the component actually performing the sensing, etc.).

It will be apparent to those skilled in the art that various modifications and variations can be made in the disclosed devices and systems without departing from the scope of the disclosure. Other aspects of the disclosure will be apparent to those skilled in the art from consideration of the specification and practice of the features disclosed herein. It is intended that the specification and examples be considered as exemplary only. Many additional variations and modifications are possible and are understood to fall within the framework of the disclosure.

## Claims

1.  A power generation device for harvesting energy through a fluid-solid triboelectric nanogenerator from a flow of body fluid flowing through or past the power generation device,
    the power generation device comprising:

    a solid component, on which surface charges are inducible,
    at least one conductive component is comprised by the solid component or fixed to the solid component, and

an output unit for outputting power,
wherein the at least one conductive component is electrically connected to the output unit, and
the solid component is configured to be brought into contact with a body fluid flowing through or past the power generation device by implanting the power generation device within a living organism or by bringing the power generation device into contact with or into proximity of a living organism.

2. The power generation device of claim 1, wherein the solid component comprises at least one polymer and/or at least one metal;
wherein the solid component optionally comprises one or several materials selected from the group consisting of: expanded polytetrafluoroethylene (ePTFE), polyethylene terephthalate, poly(L-lactic acid) (PLLA), poly-2-hydroxyethyl methacrylate graphene oxide (pHEMA/GO), polyethylene glycol graphene oxide (PEG/GO), other polymers, such as tubular polymers, silicon, polyurethane, metals, such as titanium, aluminum, gold, platinum, nickel, copper and/or stainless steel, and metal alloys, such as nitinol, cobalt-chromium alloys, magnesium alloys, brass.

3. The power generation device of claim 1 or 2, wherein the solid component comprises a lumen for letting a body fluid flow therethrough and an inside surface facing the lumen on which charges are inducible.

4. The power generation device of any one of the preceding claims, wherein at least a part of the solid component is hollow and configured to let a body fluid pass therethrough.

5. The power generation device of claim 4, comprising a part that is at least partially tube-shaped.

6. The power generation device of claim 5, wherein an inner diameter of the at least partially tube-shaped part is in the range of from 0.1 mm to 40 mm, optionally, from 0.5 mm to 30 mm, from 1 mm to 20 mm, from 1.2 mm to 10 mm, or from 1.5 mm to 6.0 mm.

7. The power generation device of claim 4 or 5, wherein a wall thickness of the at least partially tube-shaped part is in a range of from 10 $\mu$m to 10 mm, optionally, from 15 $\mu$m to 1000 $\mu$m, from 50 $\mu$m to 700 $\mu$m, or from 70 $\mu$m to 600 $\mu$m.

8. A power generation device for harvesting energy through a fluid-solid triboelectric nanogenerator from a flow of body fluid flowing through or past the power generation device,
the power generation device comprising:

at least one conductive component configured to be brought into contact with a solid component that is in contact with a body fluid flowing through or past the power generation device by implanting the power generation device within a living organism or by bringing the power generation device into contact with or into proximity of a living organism, and
an output unit for outputting power,
wherein the at least one conductive component is electrically connected to the output unit.

9. The power generation device of any one of the preceding claims, wherein the at least one conductive component is configured to be partially or fully wrapped around the solid component, and/or physically or chemically bound to, such as painted, sprayed, deep coated, sputtered, or glued onto the solid component.

10. The power generation device of any one of the preceding claims, wherein the at least one conductive component is configured to be directly or indirectly exposed to the body fluid flowing through or past the power generation device upon the power generation device being implanted within a living organism or being brought into contact with or into proximity of a living organism.

11. The power generation device of any one of the preceding claims, comprising a conductive wire, wherein the conductive wire electrically connects the at least one conductive component and the output unit.

12. The power generation device of any one of the preceding claims, wherein the at least one conductive component comprises at least one element selected from the group consisting of: silver, carbon-based materials, such as graphite, graphene, or carbon nanotubes, PEDOT:PSS (poly(3,4-ethylenedioxythiophene):polystyrene sulfonate), copper, magnesium, silver nanoparticles, platinum, gold, tantalum, titanium, silicone, iridium, iridium oxide, indium tin oxide, niobium, yttrium barium copper oxide, and magnesium diboride, bronze, brass, aluminum, iron and tungsten.

13. The power generation device of any one of the preceding claims, wherein the at least one conductive component has a length in a range of from 10 nm to 50 cm, optionally, 100 nm to 50 cm, 1 μm to 50 cm, 10 μm to 20 cm, 0.5 cm to 10 cm, or 1 cm to 9 cm.

14. The power generation device of any one of the preceding claims, comprising a plurality of conductive elements which are configured to be directly or indirectly exposed to the body fluid flowing through or past the power generation device and which are electrically connected to the output unit.

15. A self-powered sensing device comprising the power generation device of any one of the preceding claims.

16. The self-powered sensing device of claim 15, the self-powered sensing device being configured to be implanted in, on, or around a blood vessel, a fistula, a bronchial tube, intestines, nephrons, a ureter, an Eustachian tube, and/or a Fallopian tube.

17. The self-powered sensing device of claim 15 or 16, the self-powered sensing device being configured to transmit data, optionally, to wirelessly transmit data, to an external device.

18. The self-powered sensing device of any one of claims 15 to 17, the self-powered sensing device being configured to execute a function in response to sensing a predetermined signal, the function optionally being releasing a drug.

19. A system comprising the self-powered sensing device of any one of claims 15 to 18, wherein the system is configured to measure a potential difference between a first potential measured at a first point in time and a second potential measured at a second point in time, and
the system is configured to detect a flow obstruction, such as a blood clot, biofilms and encrustation, in function of the potential difference, optionally depending on whether the potential difference exceeds a first preset threshold value.

20. A system comprising the self-powered sensing device of any one of claims 15 to 19, wherein the system is configured to measure a potential difference between a first electrode and a second potential measured at a second electrode, and/or measure a current flowing, voltage or a power density of a current flowing between the first electrode and the second electrode, and
the system is configured to detect a flow obstruction, such as a blood clot, biofilms and encrustation, in function of the potential difference, optionally depending on whether the potential difference exceeds a first preset threshold value, and/or in function of whether a difference between a potential difference at a first point in time and a potential difference at a second point in time exceeds a second preset threshold value.

21. A wearable or implantable medical device comprising the power generation device according to any one of claims 1-14 or the self-powered sensing device of any one of claims 15-20,
the wearable or implantable medical device is selected from the following list: a catheter, an endovascular stent, a tubular medical device, a drainage tubes, a chest tube, an abdominal drain, a feeding tube, a ventricular shunt, a vascular graft, a decellularized vessel, an autologous graft, a xenograft, an allograft, a collagen-based graft, a decellularized organ, and a component of an extracorporeal circuit machine, such as a tubing.

22. The wearable or implantable medical device of claim 21, comprising a consumption unit, wherein the output unit is connected to the consumption unit, and
the consumption unit is selected from the following list: a sensor, a medical electronic device, such as a cardiac electronic device, a pacemaker, an implantable cardioverter defibrillator, a cardiac resynchronization therapy device, an artificial heart, an artificial organ, and a left ventricular assist device.

23. A wearable or implantable medical device of claim 22, wherein the wearable or implantable medical device is an implantable vascular graft comprising a tubular body, the tubular body being the solid component.

24. Use of the power generation device of any one of claims 1 to 14, for outputting electrical power and/or as a sensing device by monitoring a power output by the power output unit.

25. Use of the power generation device of claim 24, wherein the monitoring of the power output comprises determining a variation of flow of the body fluid over time or between at least two locations or an adsorption of one or several biocomponents, such as a blood clot, a biofilm, bacteria, or proteins, along the power generation device.

26. Use of the power generation device of claim 24 or 25 for sensing a variation in a flow of a body fluid such as blood, urine, filtered blood, lymphatic fluid, cerebrospinal fluid, serum, bile, blood plasma, saliva, synovial fluid, menstrual fluid, or tear fluid.

27. Use of the power generation device of any one of claims 1-14 for powering a wearable or implantable electronic medical device.

1

100

10

30

20

Fig. 1

10

100

200

Fig. 2

Fig. 3A

10

100

200

40

Fig. 3B

110

200

Fig. 4

Fig. 5A

Fig. 5B

Fig. 6

Fig. 7

Fig. 8

Fig. 9

Fig. 10

Fig. 11

Fig. 12

Fig. 13A

Fig. 13B

Europäisches Patentamt

European Patent Office

Office européen des brevets

## EUROPEAN SEARCH REPORT

**Application Number**

EP 25 39 8001

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 835 224 A (BEIJING INST NANOENERGY & NANOSYSTEMS) 27 October 2020 (2020-10-27) | 1,8,15, 24 | INV. A61N1/378 H02N1/04 |
| A | * paragraphs [0001], [0064], [0081] * * figures 3,7 * | 2-7, 9-14, 16-23, 25-27 | |
| | ----- | | |
| A | WANG DONGFANG ET AL: "Expanded polytetrafluoroethylene/poly(3-hydroxybutyrate) (ePTFE/PHB) triboelectric nanogenerators and their potential applications as self-powered and sensing vascular grafts", CHEMICAL ENGENEERING JOURNAL, ELSEVIER, AMSTERDAM, NL, vol. 455, 21 November 2022 (2022-11-21), XP087259087, ISSN: 1385-8947, DOI: 10.1016/J.CEJ.2022.140494 [retrieved on 2022-11-21] * Paragraph "3.4. Application of ePTFE/PHB VG-TENG in blood vascular signal monitoring" * * figure 6 * | 1-27 | |
| | ----- | | |

**TECHNICAL FIELDS SEARCHED (IPC)**

A61N
H02N

The present search report has been drawn up for all claims

| Place of search | Date of completion of the search | Examiner |
|---|---|---|
| Berlin | 9 May 2025 | Foussier, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 39 8001

This annex lists the patent family members relating to the patent documents cited in the above-mentioned European search report.
The members are as contained in the European Patent Office EDP file on
The European Patent Office is in no way liable for these particulars which are merely given for the purpose of information.

09-05-2025

| Patent document cited in search report | Publication date | Patent family member(s) | Publication date |
|---|---|---|---|
| CN 111835224 A | 27-10-2020 | NONE | |

For more details about this annex : see Official Journal of the European Patent Office, No. 12/82

EPO FORM P0459